(19) Europäisches Patentamt
European Patent Office
Office européen des brevets

(11) Veröffentlichungsnummer: **0 505 017 A2**

(12) **EUROPÄISCHE PATENTANMELDUNG**

(21) Anmeldenummer: **92250050.9**

(22) Anmeldetag: **05.03.92**

(51) Int. Cl.[5]: **C07D 471/04**, //(C07D471/04, 221:00,209:00)

(30) Priorität: **19.03.91 DE 4109342**

(43) Veröffentlichungstag der Anmeldung:
**23.09.92 Patentblatt 92/39**

(84) Benannte Vertragsstaaten:
**AT BE CH DE DK ES FR GB GR IT LI LU NL PT SE**

(71) Anmelder: **SCHERING AKTIENGESELLSCHAFT**
**Müllerstrasse 170/178**
**W-1000 Berlin 65(DE)**

(72) Erfinder: **Haffer, Gregor, Dr.**
**Celsiusstrasse 42 A**
**W-1000 Berlin 45(DE)**
Erfinder: **Börner, Helmut**
**Zugdamer Steig 23**
**W-1000 Berlin 27(DE)**
Erfinder: **Nickisch, Klaus, Dr.**
**Zescherstrasse 14**
**W-1000 Berlin 49(DE)**
Erfinder: **Deutsch, Julius, Dr.**
**Horstweg 25**
**W-1000 Berlin 19(DE)**

(54) **Selektive Phenylierung von 5-Hydroxy-beta-carbolinderivaten.**

(57) Chemoselektive Phenylierung von 5-Hydroxy-$\beta$-carbolinen in Gegenwart von schwachen Basen unter Zusatz von Wasser.

EP 0 505 017 A2

Die Erfindung betrifft das Verfahren zur Herstellung von gegebenenfalls substituierten 5-Phenoxy-β-carbolinen.

Auf Grund ihrer Affinität an die Benzodiazepin-Rezeptoren werden β-Carboline als Arzneimittel mit psychotroper Wirkung verwendet. Von den bekannten β-Carbolinen zeigen 5-Phenoxy-β-carboline, die beispielsweise in EP-A-130 140 und EP-A-234 173 beschrieben sind, sehr gute Wirksamkeit, so daß die technische Synthese dieser Verbindungen von großem Interesse ist.

Das in EP-A-234 173 beschriebene einstufige Verfahren erscheint am ehesten für die Synthese der 5-Phenoxy-β-carboline geeignet. Hierbei werden Fluorbenzolderivate in basischem Milieu in dipolaren aprotischen Lösungsmitteln, gegebenenfalls auch in Gegenwart von Phasentransferkatalysatoren umgesetzt. Mit den als Basen genannten Alkalihydroxiden und Alkalicarbonaten gelingt die Phenylierung jedoch nur mit 70% iger Ausbeute, da unter den Reaktionsbedingen auch Substitution des β-Carbolins in 9-Stellung eintritt. Dieses diphenylierte Nebenprodukt, das in 30%iger Ausbeute anfällt, kann aus dem Umsetzungsprodukt nur durch äußerst aufwendige chromatographische Trennoperationen entfernt werden und muß anschließend verworfen werden.

Es war daher wünschenswert, ein Verfahren zu entwickeln, das in technischem Maßstab und in guten Ausbeuten die Synthese der Arzneimittelwirkstoffe ermöglicht.

Es wurde überraschenderweise gefunden, daß durch Wahl einer geeigneten Base und durch Zusatz von Wasser zum aprotischen Lösungsmittel die Bildung des O,N-diarylierten Produkts fast vollständig unterdrückt und das gewünschte Produkt in über 90% iger Ausbeute durch einfache Kristallisation erhalten wird.

Dieses Ergebnis ist für den Fachmann auch besonders deshalb überraschend, weil die Hydrolyse des Fluornitrobenzols unter alkalischen Bedingungen bei Raumtemperatur und erst recht bei erhöhter Temperatur allgemein bekannt ist.

Die Erfindung betrifft das Verfahren zur Herstellung von Verbindungen der Formel I

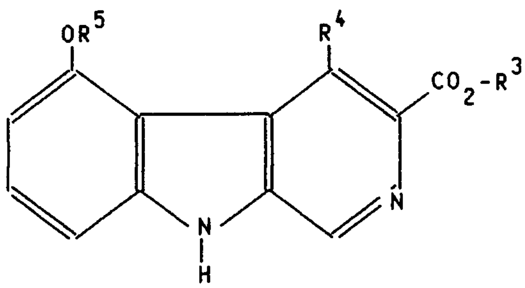

I

worin

$R^3$ $C_{1-6}$-Alkyl,

$R^4$ Wasserstoff, $C_{1-4}$-Alkyl, $C_{1-4}$-Alkoxy-$C_{1-2}$-Alkyl und

$R^5$ gegebenenfalls 1 - 2 fach substituiertes Phenyl ist, dadurch gekennzeichnet, daß man eine Verbindung der Formel II

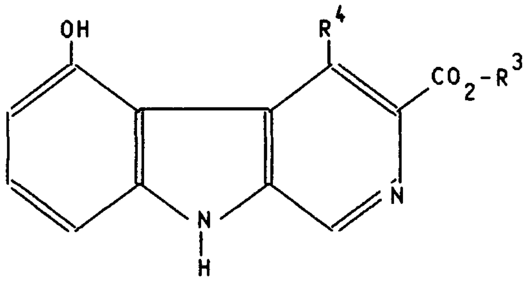

II

worin

$R^3$ und $R^4$ die obige Bedeutung haben, mit $R^5$-F der Formel III, worin $R^5$ die obige Bedeutung hat, in Gegenwart einer Base unter Zusatz von Wasser umsetzt.

Die Umsetzung wird bei erhöhten Temperaturen, von ca. 80° - 110°C in aprotischen dipolaren Lösungsmitteln wie Dimethylformamid, Dimethylacetamid, Dimethylsulfoxid, Hexamethylphosphorsäuretriamid, N-Methylpyrrolidinon, Tetramethylharnstoff, vorgenommen.

Die Reaktion ist im allgemeinen nach 5 - 9 Stunden beendet.

Als Basen für die erfindungsgemäße Umsetzung sind insbesondere schwache Basen geeignet, die die Bildung des Phenolat-Anions ermöglichen wie beispielsweise Alkalihydrogencarbonate oder Lithiumhydroxid.

Erfindungsgemäß wird Wasser in ca. 10 - 20 Volumenprozent bezogen auf das verwendete Lösungsmittel zugesetzt.

Unter Alkyl ist jeweils ein geradkettiger oder verzweigter Alkylrest zu verstehen wie beispielsweise Methyl, Ethyl, n-Propyl, Isopropyl, n-Butyl, Isobutyl, sek. Butyl, tert. Butyl, Pentyl, Isopentyl, Hexyl u.a., vorzugsweise mit bis zu 4 Kohlenstoffatomen.

Die Substituenten des Phenylrestes können 1 - 2fach in jeder beliebigen Position stehen, wobei die 2- und/oder 4-Stellung bevorzugt ist.

Als Substituent des Phenylrestes ist insbesondere die $NO_2$-Gruppe geeignet sowie die folgenden Gruppen auch als Zweitsubstituenten: Halogene wie Fluor, Chlor, Brom und Jod, Cyano, Trifluormethyl, $C_{1-4}$-Alkoxy und $C_{1-4}$-Alkyl.

Die erfindungsgemäß hergestellten Nitrophenoxy-β-carbolin-Derivate können analog den in EP-A-234 173 beschriebenen Methoden weiterverarbeit werden.

Die nachfolgenden Beispiele sollen das erfindungsgemäße Verfahren erläutern:

**Beispiel 1**

Eine Lösung von 15,7 g 5-Hydroxy-4-methoxymethyl-β-carbolin-3-isopropylester in 223 ml DMF und 34,5 ml Wasser wird mit 13,2 Kaliumhydrogencarbonat und 7 ml 4-Fluornitrobenzol versetzt und für 6 Stunden auf 90 °C erwärmt. Anschließend wird im Vakuum bei ca. 70 °C Badtemperatur auf ca. 40 ml eingeengt, der ölige Rückstand mit 100 ml Toluol verdünnt, 200 ml Wasser zugegeben, 30 Minuten nachgerührt und im Eisbad zur Kristallisation gebracht. Dann werden die ausgefallenen Kristalle abgesaugt, zweimal mit 40 ml eiskaltem Toluol und zweimal mit 40 ml Wasser nachgewaschen und getrocknet. Man erhält 19,65 g 4-Methoxymethyl-5-(4-nitrophenoxy)-β-carbolin-3-carbonsäureisopropylester vom Schmelzpunkt 217 - 219 °C.

**Beispiel 2**

3,0 g 5-Hydroxy-4-methoxymethyl-β-carbolin-3-carbonsäureethylester in 45 ml ml Dimethylformamid gelöst, versetzt man mit 6,75 ml Wasser, fügt nacheinander 2,22 g Natriumhydrogencarbonat und 1,86 g 4-Fluornitrobenzol hinzu und erhitzt 7 Stunden auf 95 °C. Nach entsprechender Aufarbeitung, wie in Beispiel 1 angegeben, werden 3,98 g 4-Methoxymethyl-5-(4-nitrophenoxy)-β-carbolin-3-carbonsäureethylester vom Schmelzpunkt 231 - 232 °C erhalten.

**Patentansprüche**

**1.** Verfahren zur Herstellung einer Verbindung der Formel I

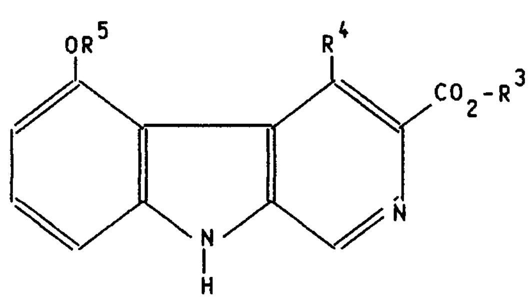

I

worin

$R^3$ $C_{1-6}$-Alkyl,

$R^4$ Wasserstoff, $C_{1-4}$-Alkyl, $C_{1-4}$-Alkoxy-$C_{1-2}$-Alkyl und

$R^5$ einen gegebenenfalls 1 - 2 fach substituierten Phenylrest darstellt,

dadurch gekennzeichnet, daß man eine Verbindung der Formel II

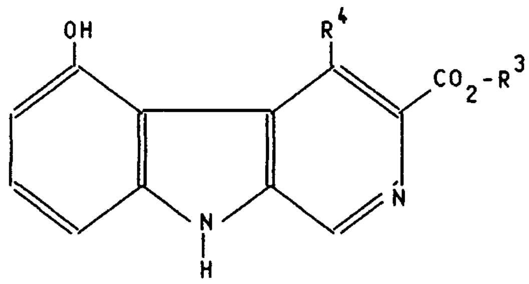

II

worin

$R^3$ und $R^4$ die obige Bedeutung haben, mit $R^5$-F der Formel III, worin $R^5$ die obige Bedeutung hat, in Gegenwart einer Base unter Zusatz von Wasser umsetzt.

**2.** Verfahren nach Anspruch 1, dadurch gekennzeichnet, daß die Umsetzung in Gegenwart von schwachen Basen vorgenommen wird.